# EUROPEAN PATENT SPECIFICATION

(11) **EP 1 392 308 B1**
(45) Date of publication and mention of the grant of the patent: **05.04.2006**
(21) Application number: 02728820.8
(22) Date of filing: 17.04.2002
(51) Int. Cl.: A61K 31/495, A61K 31/19, A61K 31/20, A61K 31/557, A61P 27/06

(54) **OPHTHALMICALLY ACCEPTABLE COMPOSITIONS COMPRISING AN ALPHA-2-ADRENERGIC AGONIST AND A FATTY ACID**
ZUSAMMENSETZUNGEN ENTHALTEND EINEN ALPHA-2-ADRENOREZEPTORAGONISTEN UND EINE FETTSAEURE
COMPOSITIONS OPHTHALMIQUES COMPRENANT UN AGONISTE ALPHA-2-ADRENERGIQUE ET UN ACIDE GRAS

(30) Priority: 03.05.2001 US 848249
(43) Date of publication of application: 03.03.2004
(73) Proprietor: ALLERGAN, INC., Irvine, California 92612 (US)
(72) Inventor: WOODWARD, David, F., Lake Forest, CA 92630 (US); AMBRUS, Gyorgy, Santa Ana, CA 92705 (US)
(74) Representative: HOFFMANN EITLE
(86) International application number: PCT/US2002/012219
(87) International publication number: WO 2002/089804

(56) References cited:
- EP-A- 0 305 726
- EP-A- 0 426 390
- WO-A-00/44355
- US-A- 4 188 393
- US-A- 4 629 621
- US-A- 5 811 443
- PATENT ABSTRACTS OF JAPAN vol. 1999, no. 10, 31 August 1999 (1999-08-31) & JP 11 130656 A (KOSE CORP), 18 May 1999 (1999-05-18)

## Description

### Background of the Invention

The present invention relates to compositions containing alpha-2-adrenergic agonists (hereinafter) "agonists"). More particularly, the invention relates to such compositions in which the agonists have enhanced pharmacokinetic characteristics. An agonist of this invention comprises a quinoxaline compound.

A continuing challenge in providing compositions containing agonists is to be able to render such agonists more effective. One technique of making such agonists more effective is by enhancing their pharmacokinetic disposition. For example, the dispensed or administered agonists should advantageously be permeable through lipid cell membranes so that the agonists may reach the target receptor to impart a therapeutic effect. One possible reason for why certain agonists permeate poorly through a lipid membrane is that these agonists may be charged ions at physiological pH.

Although the term "enhancement of pharmacokinetic disposition" as used herein, may mean an enhancement in permeability, an enhancement of pharmacokinetic disposition may also mean an enhancement in, for example, bioavailablity, sequestration and release characteristics of the agonists.

Ion pairing, or complexation, between cations and anions to enhance the movement of ionizable molecules across biologic membranes have been suggested. Nash et al. *Skin Pharmacol* 5:160-170 (1992) and Ogawa et al. *Jpn J Ophthalmol* 37:47-55 (1993) . However, prior ion complex systems may be inappropriate for use to deliver agonists to certain biological environments, for example the ophthalmic environment.

WO 98/02187 discloses a pharmaceutical composition containing an acid addition salt of a basic drug and a fatty acid or bile acid. The acid addition salts thus formed exhibit enhanced transmucosal and transdermal penetration of the basic drug. The acid addition salts, an inclusion complex containing said salts and a use of said salts are also disclosed in WO 98/02187. EP 0 426 390 discloses topical ophthalmic formulations of quinoxalines.

There continues to be a need for new compositions that increase the efficacy of alpha-2-adrenergic agonists.

### Summary of the Invention

New agonist-containing compositions have been discovered. In accordance with the invention, the present compositions contain certain materials which are effective in enhancing the efficacy of the agonists of the compositions. Without limiting the invention to any particular theory or mechanism of operation, it is believed that the efficacy of the agonists is enhanced because of improved pharmacokinetics, for example, increased permeability of the agonists through lipid bilayers. In one embodiment, these materials enhance the bioavailability of the agonists in the eye. Preferably, the materials are able to enhance the pharmacokinetics of the agonists under physiological conditions, for example at pH's of about 7 to about 9.

further in accordance with the invention, the agonists are ionized at physiological pH's, for example 6.5 to 9.0. In one embodiment, the agonists are ionized at about pH 7.

Still further in accordance with the invention, agonists employed in the present compositions include those compounds, mixtures of compounds, mixtures of other materials, which are useful to provide a therapeutic benefit or effect when administered to a patient, e.g. a human patient. The agonists useful in this invention comprise quinoxaline components. Quinoxaline components include quinoxaline, biologically compatible salts thereof, esters thereof, other derivatives thereof and the like, and mixtures thereof. Non-limiting examples of quinoxaline derivatives include (2-imidozolin-2-ylamino) quinoxaline, 5-bromo-6-(2-imidozolin-2-ylamino) quinoxaline (hereinafter "bromonidine"), biologically compatible salts thereof and esters thereof.

Still further in accordance with the invention, the agonists are specific for the alpha-2A-adrenergic receptors, alpha-2B-adrenergic receptors and/or alpha-2D-adrenergic receptors or any combination thereof.

Still further in accordance with the invention, materials which enhance the pharmacokinetics of the agonists are fatty acid components, hereinafter FACs. The FAC are fatty acids, saturated and/or unsaturated. The fatty acids of the present invention may have more than 12 carbons, for example docosahexanoic acid and linolenic acid. In one embodiment, the fatty acids of the present invention comprise about 12 to about 26 carbons. In another embodiment, the fatty acids of the present invention comprise about 16 to about 24 carbons. In one embodiment, the FACs themselves are effective to provide at least one therapeutic effect.

Still further in accordance with the invention, the agonist and the FAC forms a complex. In one embodiment, the agonist and the FAC forms a complex in solution, preferably a solution at pH's of about 7 to about 9. In one embodiment, the agonist and FAC are able to form a complex, for example a salt, outside a solution.

Still further in accordance with the invention, the compositions include carrier components. In one embodiment, the compositions have pH's of about 7 or greater, preferably about 7 to about 9, and are ophthalmically acceptable.

Additional advantages and aspects of the present invention are apparent in the following detailed description and claims.

### Detailed Description of the Invention

Compositions comprising alpha-2-adrenergic agonists (agonists) and fatty acid components (FACs) are provided. The FACs employed in the present compositions may be effective in enhancing the pharmacokinetics of the agonists. For example, the FAC may enhance the therapeutic effect of the agonist. In one embodiment, the present compositions may further include liquid carrier components and have the characteristics of liquid, for example, aqueous liquid, solutions.

In one embodiment, the agonist and the FAC form complexes. The complexes formed may be a "loose" ion pair or a "tight" ion pair. Preferably, the complex of the present invention is a "tight" ion pair. More preferably, the complexes of this invention are adequately "tight" as to not dissociate in high dielectric constant solvent, such as water or aqueous solutions. One advantage of such a "tight" ion pair complex is that the complex may be contained in an aqueous solution, for example saline, which may be used in an ophthalmic environment. However, it is also preferable that the complex is able to dissociate under certain conditions. For example, after the complex crosses the lipid layer, the agonist may activate a targeted molecule more effectively if it is not complexed to a FAC. Therefore, in a preferred embodiment, the agonist and the FAC exist as a complex for the purpose of enhancing the pharmacokinetic disposition of the agonist and thereafter dissociate to allow the agonist to act more effectively at a receptor.

In one embodiment, a single agonist may form a complex with more than one FAC, for example two or three FACs. In another embodiment, a single FAC may form a complex with more than one agonist, for example two or three agonists.

The presently useful agonists preferably are chosen to benefit from the presence of the FACs. In general, the agonists are provided with enhanced ability to cross a lipid membrane when they complex with the FACs. In one embodiment, the agonists are basic molecules. In another embodiment, the agonists are cations.

In a preferred embodiment, the useful agonists include molecules containing amines. Also, the adrenergic agonists preferably are amine-containing molecules with pKa's of greater than 7, preferably about 7 to about 9.

As used herein, the term "alpha-2 adrenergic agonist" or "agonist" includes chemical entities, such as compounds, ions, complexes and the like, that produces a net sympatholytic response, resulting in increased accommodation, for example, by binding to presynaptic alpha-2 receptors on sympathetic postganglionic nerve endings or, for example, to postsynaptic alpha-2 receptors on smooth muscle cells. A sympatholytic response is characterized by the inhibition, diminishment, or prevention of the effects of impulses conveyed by the sympathetic nervous system. The alpha-2 adrenergic agonists of the invention bind to the alpha-2 adrenergic receptors presynaptically, causing negative feedback to decrease the release of neuronal norepinephrine. Additionally, they also work on alpha-2 adrenergic receptors postsynaptically, inhibiting beta-adrenergic receptor-stimulated formation of cyclic AMP, which contributes to the relaxation of the ciliary muscle, in addition to the effects of postsynaptic alpha-2 adrenergic receptors on other intracellular pathways. Activity at either pre- or postsynaptic alpha-2 adrenergic receptors will result in a decreased adrenergic influence. Decreased adrenergic influence results in increased contraction resulting from cholinergic innervations. Alpha-2 adrenergic agonists also include compounds that have neuroprotective activity. For example, 5-bromo-6-(2-imidozolin-2-ylamino) quinoxaline is an alpha-2-adrenergic agonist which has a neuroprotective activity through an unknown mechanism.

The alpha 2 adrenergic agonists useful in this invention are quinoxaline components. In one embodiment, the quinoxaline components include quinoxaline, derivatives thereof and mixtures thereof. Preferably, the derivatives of quinoxaline include (2-imidozolin-2-ylamino) quinoxaline. More preferably, the derivatives of quinoxaline include 5-halide-6-(2-imidozolin-2-ylamino) quinoxaline. The "halide" of the 5-halide-6-(2-imidozolin -2-ylamino) quinoxaline may be a fluorine, a chlorine, an iodine, or preferably, a bromine, to form 5-bromo-6-(2-imidozolin-2-ylamino) quinoxaline.

Other useful quinoxaline derivatives are well known. For example, useful derivatives of a quinoxaline include the ones disclosed by Burke et al U.S. Patent No. 5, 703, 077. See also Danielwicz et al 3, 890, 319 . Each of the disclosures of Burke et al and Danielwicz et al is incorporated in its entirety by reference herein.

The quinoxaline and derivatives thereof, for example Brimonidine, are amine-containing and preferably have pKa's of greater than 7, preferably about 7.5 to 9. Analogs of the foregoing compounds that function as alpha-2 adrenergic agonists also are specifically intended to be embraced by the invention.

Preferably, the agonists, for example the ones listed above, are effective toward activating one or more of alpha-2A-adrenergic receptors, alpha-2B-adrenergic receptors and alpha-2D-adrenergic receptors.

In one useful embodiment, the amount of agonist in the present composition is in the range of about 0.05% to about 30% (w/v) or more of the composition. Preferably, the amount of agonist is in the range of about 0.1% (w/v) to about 10% (w/v). More preferably, the amount of agonist is in the range of about 0.1% (w/v) to about 0.6% (w/v). Even more preferably, the agonist is bromonidine and is present in the composition in the range of about 0.1% (w/v) to about 0.6% (w/v), preferably about 0.13%.

Any suitable FACs may be employed in accordance with the present invention. The FACs are fatty acids Preferably, the fatty acids possess a long hydrophobic carbon chain and a terminal carboxyl group. The chain may be saturated, or it may have one or more double bonds. Moreover, a few fatty acids contain triple bonds. Fatty acids differ primarily in length and in the number and position of their unsaturated bonds. Non-limiting examples of saturated fatty acids include lauric acid, myristic, palmitic, stearic, arachidic, lignoceric and mixtures thereof. Non-limiting examples of unsaturated fatty acids include palmitoleic, oleic, linoleic, linolenic, arachidonic and mixtures thereof. Other examples of some unusual fatty acids include trans-Vaccenic , acid, lactobacillic, tuberculostearic, cerebronic, and mixtures thereof.

In a preferred embodiment, the FAC includes docosahexanoic acids. In another preferred embodiment, the FAC includes linolenic acids.

In one embodiment, the fatty acids of the present invention comprise about 12 to about 26 carbon atoms. In a preferred embodiment the fatty acids of the present invention comprises about 16 to about 24 carbons.

In a preferred embodiment, the FAC has a direct therapeutic effect. For example, a FAC may include eicosanoids such as prostanoids. A prostanoid is any group of complex fatty acids derived from arachidonic acid, being 20 carbons in length with an internal 5 or 6-carbon ring, for example prostaglandin, protanoic acid and thromboxanes. Prostanoids are known to reduce intra-ocular pressure. In a preferred embodiment, a composition according to the invention comprises a complex having an agonist and a therapeutically effective FAC. For example, a composition according to the present invention may comprise a complex of an adrenergic agonist and a prostanoid. Both the adrenergic agonist and the prostanoid may act, via different mechanisms, to provide an additive therapeutic effect, for example, to reduce intra-ocular pressure. The FAC may exert its therapeutic effects when it is still bound to a complex, or the EEC may exert its effects when it is free from the complex.

In one embodiment, a complex of an agonist and a FAC may exist as a salt outside of a solution. For example, a complex of brimonidine and linoleic acid may be a powder. Furthermore, this complex may be added to a solution, for example a saline solution. Preferably, the agonist and the FAC still remain as a complex. In one embodiment, the solution containing the complex, for example a complex of bromonidine and linoleic acid, is administered to the eye to treat glaucoma. In one embodiment, the complex remains intact at the site where the agonist may exert a therapeutic effect. In a preferred embodiment, the complex dissociates at or near the site where the alpha-2-adrenergic agonist may exert a therapeutic effect. For example, a complex of bromonidine linolenic acid may dissociate to release bromonidine at or near the ciliary body in the eye, wherein the bromonidine can act on receptors located on the ciliary body to reduce the production of aqueous solutions, thereby treating glaucoma.

In another embodiment, a FAC is added to a solution containing an agonist to form a complex with the agonist therein. In one embodiment, the complex is formed only in solution. The amount of FAC added is such that the pharmacokinetics of the agonist is at least somewhat enhanced. Such amount should be effective to perform the desired function or functions in the present composition and/or after administration to the human or animal. In one embodiment, the amount of FAC is sufficient to complex at least in a major amount, and more preferably substantially all, of the agonists in a solution of the present composition. In one useful embodiment, the amount of FAC in the present composition is in the range of about 0.05% to about 30% (w/v) or more of the composition. Preferably, the amount of FAC is in the range of about 0.1% (w/v) to about 10% (w/v). More preferably, the amount of FAC is in the range of about 0.1% (w/v) to about 0.6% (w/v). Even more preferably, the FAC is docosahexanoic acid or linolenic acid and is present in the composition in the range of about 0.1% (w/v) to about 0.6% (w/v). A particularly useful concentration of FAC, for example linoleic acid, in the present compositions is about 0.12%.

In one embodiment, the agonists and the FACs form complexes at pH's of greater than 7. Preferably, the agonists and the FACs form complexes at pH's between about 7 to about 10.

In one embodiment, the complex according to the present invention may serve as a delay release system for the agonists and/or the FACs. For example, an agonist may be pharmacologically inactive when it is part of a complex. However, as the complex slowly dissociates over time in a biological environment, it slowly releases the agonist. The slow or delayed release of a pharmacologically active agonist may be advantageous. For example, such delayed release may be helpful in providing appropriate dosing.

In one embodiment, the complexation of agonists with FACs further help solubilize the agonists in solution and preferably reduces irritation when the agonists are administered to sensitive tissues. For example, an eye drop solution having a pH of about 7 may contain insoluble agonist ions, such as bromonidine tartrate ions. If such a solution is administered to the eye, a sensitive tissue, the insoluble agonist ions may cause discomfort and irritation. However, a complex of agonist and FAC may help solubilize the agonist in such a solution. In a preferred embodiment, the solution containing a solubilized agonist results in less irritation as the solution is applied to a sensitive tissue, for example the eye. In a more preferred embodiment, the solution containing solubilized agonist results in little or no irritation when the solution is administered to a sensitive tissue.

In one embodiment, the compositions may also include preservative components or components which assist in the preservation of the composition. The preservative components selected so as to be effective and efficacious as preservatives in the present compositions, that is in the presence of FACs, and preferably have reduced toxicity and more preferably substantially no toxicity when the compositions are administered to a human or animal.

Preservatives or components which assist in the preservation of the composition which are commonly used in pharmaceutical compositions are often less effective when used in the presence of solubilizing agents. In certain instances, this reduced preservative efficacy can be compensated for by using increased amounts of the preservative. However, where sensitive or delicate body tissue is involved, this approach may not be available since the preservative itself may cause some adverse reaction or sensitivity in the human or animal, to whom the composition is administered.

Preferably, the present preservative components or components which assist in the preservation of the composition, preferably the agonists therein, are effective in concentrations of less than about 1% (w/v) or about 0. 8 0 (w/v) and may be 500 ppm (w/v) or less, for example, in the range of about 10 ppm(w/v) or less to about 200 ppm(w/v). Preservative components in accordance with the present invention preferably include, but are not limited to, those which form complexes with the anionic polymer to a lesser extent than does benzalkonium chloride.

Very useful examples of the present preservative components include, but are not limited to oxidative preservative components, for example oxy-chloro components, peroxides, persalts, peracids, and the like, and mixtures thereof. Specific examples of oxy-chloro components useful as preservatives in accordance with the present invention include hypochlorite components, for example hypochlorites; chlorate components, for example chlorates; perchlorate components, for example perchlorates; and chlorite components. Examples of chlorite components include stabilized chlorine dioxide (SCD), metal chlorites, such as alkali metal and alkaline earth metal chlorites, and the like and mixtures thereof. Technical grade (or USP grade) sodium chlorite is a very useful preservative component. The exact chemical composition of many chlorite components, for example, SCD, is not completely understood. The manufacture or production of certain chlorite components is described in McNicholas U.S. Patent 3,278,447. Specific examples of useful SCD products include that sold under the trademark Dura Klor® by Rio Linda Chemical Company, Inc., and that sold under the trademark Anthium Dioxide® by International Dioxide, Inc. An especially useful SCD is a product sold under the trademark Purite™ by Allergan, Inc. Other examples of oxidative preservative components include peroxy components. For example, trace amounts of peroxy components stabilized with a hydrogen peroxide stabilizer, such as diethylene triamine penta(methylene phosphonic acid) or 1-hydroxyethylidene-1,1-diphosphonic acid, may be utilized as a preservative for use in components designed to be used in the ocular environment. Also, virtually any peroxy component may be used so long as it is hydrolyzed in water to produce hydrogen peroxide. Examples of such sources of hydrogen peroxide, which provide an effective resultant amount of hydrogen peroxide, include sodium perborate decahydrate, sodium peroxide and urea peroxide. It has been found that peracetic acid, an organic peroxy compound, may not be stabilized utilizing the present system. See, for example, Martin et al U.S. Patent No. 5,725,887.

Preservatives other than oxidative preservative components may be included in the compositions. The choice of preservatives may depend on the route of administration. Preservatives suitable for compositions to be administered by one route may possess detrimental properties which preclude their administration by another route. For nasal and ophthalmic compositions, preferred preservatives include quaternary ammonium compounds, in particular the mixture of alkyl benzyl dimethyl ammonium compounds and the like known generically as "benzalkonium chloride."

In one broad embodiment, compositions are provided which comprise an agonist-FAC complex, a preservative component in an effective amount to at least aid in preserving the compositions and a liquid carrier component. Preferably, the preservative components include oxy-chloro components, such as compounds, ions, complexes and the like which (1) do not substantially or significantly detrimentally affect the agonist in the compositions or the patients to whom the compositions are administered, and (2) are substantially biologically acceptable and chemically stable. In one embodiment, compositions in accordance with the present invention comprise a complex of alpha-2-adrenergic agonist-linolenic acid, an oxy-chloro component, and a liquid carrier component.

The carrier components useful in the present invention are selected to be non-toxic and have no substantial detrimental effect on the present compositions, on the use of the compositions or on the human or animal to whom the compositions are administered. In one embodiment, the carrier component is a liquid carrier. In a preferred embodiment, the carrier component is a liquid carrier component. A particularly useful liquid carrier component is that derived from saline, for example, a conventional saline solution or a conventional buffered saline solution. The liquid carrier preferably has a pH in the range of about 6 to about 9 or about 10, more preferably about 6 to about 8, and still more preferably about 7.5. The liquid medium preferably has an ophthalmically acceptable tonicity level, for example, of at least about 200 mOsmol/kg, more preferably in the range of about 200 to about 400 mOsmol/kg. In an especially useful embodiment, the osmolality or tonicity of the carrier component substantially corresponds to the tonicity of the fluids of the eye, in particular the human eye.

In one embodiment, the carrier components containing the FACs and the agonists may have viscosities of more than about 0.01 centipoise (cps) at 25°C, preferably more than about 1 cps at 25°C, even more preferably more than about 10 cps at 25°C. In a preferred embodiment, the composition has a viscosity of about 50 cps at 25°C and comprises a conventional buffer saline solution, a docosahexanoic acid and a brimonidine.

In order to insure that the pH of the liquid carrier component, and thus the pH of the composition, is maintained within the desired range, the liquid carrier component may include at least one buffer component. Although any suitable buffer component may be employed, it is preferred to select such component so as not to produce a significant amount of chlorine dioxide or evolve significant amounts of gas, such as CO. It is preferred that the buffer component be inorganic. Alkali metal and alkaline earth metal buffer components are advantageously used in the present invention.

Any suitable ophthalmically acceptable tonicity component or components may be employed, provided that such component or components are compatible with the other ingredients of the liquid carrier component and do not have deleterious or toxic properties which could harm the human or animal to whom the present compositions are administered. Examples of useful tonicity components include sodium chloride, potassium chloride, mannitol, dextrose, glycerin, propylene glycol and mixtures thereof. In one embodiment, the tonicity component is selected from inorganic salts and mixtures thereof.

The present compositions may conveniently be presented as solutions or suspensions in aqueous liquids or non-aqueous liquids, or as oil-in-water or water-in-oil liquid emulsions. The present compositions may include one or more additional ingredients such as diluents, surface active agents, thickeners, lubricants, and the like, for example, such additional ingredients which are conventionally employed in compositions of the same general type.

The present compositions in the form of aqueous suspensions may include excipients suitable for the manufacture of aqueous suspensions. Such excipients are suspending agents, for example, sodium carboxymethylcellulose, methylcellulose, hydroxypropylmethylcellulose, sodium alginate, polyvinylpyrrolidone, gun tragacanth and gun acacia; dispersing or wetting agents may be a naturally occurring phosphatide, for example, lecithin, or condensation products of ethylene oxide with long chain aliphatic alcohols, for example, heptadecaethyleneoxycetanol, or condensation products of ethylene oxide with partial esters derived from fatty acids and a hexitol such as polyoxyethylene sorbitol mono-oleate, or condensation products of ethylene oxide with partial esters derived from fatty acids and hexitol anhydrides, for example, polyoxyethylene sorbitan mono-oleate, and the like and mixtures thereof.

The present compositions in the form of oily suspensions may be formulated in a vegetable oil, for example, olive oil, sesame oil or coconut oil, or in a mineral oil such as liquid paraffin. Such suspensions may contain a thickening agent, for example beeswax, hard paraffin or cetyl alcohol.

The present compositions may also be in the form of oil-in-water emulsions. The oily phase may be a vegetable oil, for example, olive oil or arachis oil, or a mineral oil, for example, liquid paraffin, and the like and mixtures thereof. Suitable emulsifying agents may be naturally-occurring gums, for example, gum acacia or gun tragacanth, naturally-occurring phosphatides, for example, soya bean lecithin, and esters or partial esters derived from fatty acids and hexitol anhydrides, for example, sorbitan mono-oleate, and condensation products of the said partial esters with ethylene oxide, for example, polyoxyethylene sorbitan mono-oleate.

The specific dose level for any particular human or animal depends upon a variety of factors including the activity of the active component employed, the age, body weight, general health, sex, diet, time of administration, route of administration, rate of excretion, drug combination and the severity of the particular condition undergoing therapy.

The following non-limiting examples illustrate certain aspects of the present invention.

### EXAMPLE 1

### Effects of bromonidine-linoleic acid on Intra Ocular Pressure

The data below shows the percent change with time of Intra Ocular Pressure (mm Hg) after an administration of bromonidine-linoleic acid complex at time 0. The complex is an ion pair formulation of 0.131% bromonidine and 0.126% linoleic acid.

| | |
|---|---|
| 0 hr | administration of complex |
| 1 hr | -10.4% |
| 2 hr | -16.0% |
| 4 hr | -09.5% |
| 6 hr | -09.4% |

### EXAMPLE 2

### Relative sedative effects of various compounds

The relative sedative effects of bromonidine-linoleic acid (compound 65) were compared to saline (compound 62) and Brimonidine tartrate (compound 60). This study involved cross overs and a one-week wash out in between the administration of the various compounds.

As done in a previous experiment, the following method was followed:
1. 6 trained monkeys were placed in chairs and allowed to acclimate for approximately 30 minutes.
2. Individual monkeys were brought into the "testing room" where they were allowed to adjust to the new environment for approximately 2 minutes. After this adjustment time the monkey was observed for 1-2 minutes after which a sedation score was given. Sedation scores were recorded on an observation sheet.
3. The monkey was returned to the group of animals assigned to this study.
4. 2 baseline readings were done at T-0.5 and 0 hour. After the 0 hour reading one drop of the test compound was administered to the right eye.
5. Steps 2 & 3 were repeated at T=0.5, 1, 2, 3 and 4 hour.
6. Animals were monitored until they recover from effects of the drug.
7. Scoring of Sedation was based on the following scale:
S=0 Monkey is quiet, but slightly active.
S=1 Monkey is quiet, easy to handle for reading
S=2 Monkey is quiet, relaxed but very low in activity
S=3 Monkey is blinking eyes and yawning
S=4 Monkey is sleepy and inactive, eyes are heavy
8. The test compounds were coded: 62-Saline, 65-bromonidine tartrate, 60-bromonidine-linoleic acid.
9. Test Compound Administration:

| animal #1 | week 1 | week 2 | week 3 |
|---|---|---|---|
| 19 | 62 | 65 | 60 |
| 24 | 62 | 65 | 60 |
| 42 | 65 | 60 | 62 |
| 50 | 65 | 60 | 62 |
| 57 | 60 | 62 | 65 |
| 58 | 60 | 62 | 65 |

The scoring was conducted for each animal for the different test compounds. The average results are shown on table 1.

**Table 1 Comparison of the sedative effects of Brimonidine-Linoleic Acid ion pair complex (0.2%) to Brimonidine Tartrate (0.2%) and saline.**

| Brimonidine-Linoleic Acid Ion Pair Complex | |
|---|---|
| **TIME (HR)** | **SEDATION SCORE** |
| -0.5 | 0.7 |
| 0 | 1.0 |
| 0.5 | 1.2 |
| 1 | 1.5 |
| 2 | 1.8 |
| 3 | 1.6 |
| 4 | 1.6 |

| Brimonidine Tartrate | |
|---|---|
| **TIME (HR)** | **SEDATION SCORE** |
| -0.5 | 0.7 |
| 0 | 0.8 |
| 0.5 | 0.8 |
| 1 | 1.7 |
| 2 | 2.6 |
| 3 | 2.5 |
| 4 | 2.7 |

| Saline Vehicle | |
|---|---|
| **TIME (HR)** | **SEDATION SCORE** |
| -0.5 | 0.7 |
| 0 | 1.0 |
| 0.5 | 1.0 |
| 1 | 1.0 |
| 2 | 1.2 |
| 3 | 1.3 |
| 4 | 1.3 |

The first reading after dosing, 0.5 hr-time point, the monkeys were quiet and easy to handle. In general, the animals started to show low activity when brought into the test room at the 1hr time.

Half the monkeys given the test compound bromonidine-linoleic showed low activity (1 hour post dose), with the exception of monkey #19. Monkey #19 did not appear to be sleepy, inactive or have heavy eyes and seemed to react similarly to all test compounds. She seems to be very comfortable in the chair, and when there were no distractions she tended to close her eyes and relax.

The dosing with bromonidine-linoleic acid complex appears to cause more sedation in the monkeys than dosing with saline. In general when the monkeys were dosed with saline, they were quiet and easy to handle for all readings. However, dosing with bromonidine tartrate causes more sedation than dosing with bromonidine-linoleic acid. When the monkeys were dosed with bromonidine tartrate, on average they became sleepy and inactive with heavy eyes. This observation was seen usually at the 2-hour time point and most of the animals remained this way through the end of observations.

Without wishing to limit the invention to any mechanism or theory of operation, it is believed that one of the reasons that bromonidine-linoleic acid complex causes less sedation than bromonidine tartrate is that it partitions more in the lipid compartments. In other words, the bromonidine-linoleic acid complex is more trapped in the lipid compartments, and is not as available to circulate in the blood stream to eventually travel to the brain to cause sedation.

### Example 3

### Effects of bromonidine-linoleic acid ion pair complex (0.2%) on rabbit intraocular pressure

In this study, the animals were placed into three groups consisting of a mix of age, size and sex.

| **Group Number** | **Number of Animals Males** | **Number of Animals Females** |
|---|---|---|
| 1 | 4 | 4 |
| 2 | 4 | 4 |
| 3 | 4 | 4 |

One group of animals (both sexes) was used per screening study. The test compound (20*µ*L of 0.2% bromonidine-linoleic acid ion pair complex) was administered to the surface of the cornea using an automatic pipette or an appropriate device.

The following general procedure for administering the test compound employed in this study is presented below:
1. Make sure that the 0.25% proparacaine Opthetic® mixture (topical local anesthetic), test compounds, and commercially available treats are available.
2. Turn on the Digilab Modular One™ Pneuma Tonometer (pneumatonometer) [BioRad, Cambridge, MA]; it needs approximately 15 minutes to warm up. Turn on gas supply or air pump.
3. Hold probe vertically with the point tip down and press "calibration check" for "zero".
4. Wipe the pneumatonometer probe with an alcohol swab, and inspect the tip membrane for holes.
5. Set the external calibration device (air or water manometer) to 25 mm Hg, place the pneumatonometer probe in the calibration device to ensure that it corresponded to 25± 2mm Hg.
6. Obtain a rabbit. Make sure the recording data sheets are at hand.
7. Gently restrain the rabbit via a commercial restrainer or a cotton towel. Measure the pupil diameter with the specialized ruler for each eye and record the values on the sheets provided. If the eyes are too dark to obtain a value by this method, a specialized penlight may be used. Obtain a pupil diameter measurement by shining the penlight on the cornea for one second.
8. Slide the upper eyelid up with the thumb and visually assess and score the degree of ocular surface redness. Record the value for each eye on the sheet provided.
9. Put one drop of 0.25% proparacaine Opthetic® (50:50 mix of 0.5% proparacaine Opthetic® + Cellufresh®) on the surface of each eye. Wait one or more minutes for ophthetic to take effect.
10. Place the pnematonometer tip on one eye at the site where the curvature of the cornea is greatest. Let the probe shank travel to the black line, not the red line. Persist until a stable reading with the standard deviation below 1.0 is obtained. Repeat the procedure for the contralateral eye. Record the data. (Note: If the animal is upset by the restraint, the reading will be artificially high and cannot be used. Use gentle restraint).
11. At the end of the 0, 6, 24, 30, 48, 54, 72, 78 and 96 hour measurements, use an automatic pipette to apply 20*µ*L of the test compound to the surface of the cornea of one eye. After the 102 hour measurements, the eyes will be washed out with Refresh® and Prefrin®.
12. Give the animal a treat and return the animal to its cage.
13. Repeat steps 7 to 11 on the remaining animals in the group.

The following general procedure for measuring the effects of the test compound employed in this study is as follows: a reading is conducted at 0, 2, 4, 6, 24, 26, 28, 30, 48, 50, 52, 54, 72, 74, 76, 78, 96, 98, 100, and 102 hours. The pneumatonometer is calibrated before use with a manometer and the probe tip is wiped with an alcohol swab. One drop of 0.25% proparacaine Opthetic®, a corneal anesthetic, is placed on the cornea. Allow sufficient time (approximately 1 minute) for it to anesthetize the cornea before placing the probe on the eye. The eye is gently opened by the person doing the tonometer reading. The probe is placed on the cornea at the point of greatest curvature and a stable reading is obtained. The probe is held parallel to the floor and perpendicular to the line of the rabbit's sight. The reading is repeated until a reasonable reading can be obtained. The piston should be between the red and black lines or at the black line on the probe.

The effects of bromonidine-linoleic acid ion pair complex are shown on Table 2. It appears that the complex is able to reduce intraocular pressure in a rabbit's eye for at least 6 hours. For example, 6 hours after the administration at times 0 hr, 24hr, 48hr, 72 hr, and 96 hr, the intraocular pressure remained below the initial time. However, it also appears that the effect of the complex is less than 18 hrs. For example, 18 hrs after administration of the complex at time 6 hr, the intraocular pressure returned to about the same initial level.

| **TIME (HR)** | **INTRAOCULAR PRESSURE (mmHg)** |
|---|---|
| 0* | 25.8 ± 1.2 |
| 2 | 17.1 ± 1.0 |
| 4 | 21.1 ± 1.0 |
| 6* | 23.9 ± 1.0 |
| 24* | 25.6 ± 0.5 |
| 26 | 19.9 ± 0.6 |
| 28 | 22.9 ± 0.8 |
| 30* | 23.0 ± 1.1 |
| 48* | 26.5 ± 1.0 |
| 50 | 20.4 ± 0.6 |
| 52 | 23.4 ± 0.7 |
| 54* | 24.1 ± 0.8 |
| 72* | 26.8 ± 0.9 |
| 74 | 21.1 ± 0.8 |
| 76 | 23.9 ± 1.1 |
| 78* | 25.2 ± 0.9 |
| 96* | 27.9 ± 1.1 |
| 98 | 20.1 ± 1.3 |
| 100 | 23.9 ± 0.7 |
| 102 | 125. ± 1.6 |

| | |
|---|---|
| *bromonidine-linoleic acid ion pairs were administered at these time points. | |
| Values are mean ± S.E.M. n=8. | |

While this invention has been described with respect to various specific examples and embodiments, it is to be understood that the invention is not limited thereto and that it can be variously practiced with the scope of the following claims.

## Claims

1. An ophtalmically acceptable composition comprising: an alpha-2-adrenergic agonist selected from a quinoxaline compound, and a fatty acid component selected from the group consisting of saturated fatty acids and unsaturated fatty acids, and mixtures thereof the fatty acid component forms a complex with the alpha-2-adrenergic agonist; the complex remaining substantially intact in an aqueous environment.

2. A composition of claim 1 wherein the fatty acid component is present in an amount effective to enhance the efficacy of the agonist relative to the efficacy of the alpha-2-adrenergic agonist without the fatty acid component.

3. A composition of claim 2 wherein the quinoxaline component is selected from the group consisting of quinoxaline, (2-imidozolin-2-ylamino) quinoxaline, 5-bromo-6-(2-imidozolin-2-ylamino) quinoxaline, and derivatives thereof and mixtures thereof.

4. A composition of claim 1 wherein the fatty acid component is selected from the group consisting of fatty acids having about 12 to about 26 carbon atoms per molecule, derivatives thereof and mixtures thereof.

5. A composition of claim 1 wherein the fatty acid component is selected from the group consisting of docosahexanoic acids, derivatives thereof and mixtures thereof.

6. A composition of claim 1 wherein the fatty acid component is selected from the group consisting of linolenic acids and mixtures thereof.

7. A composition of claim 1 wherein the fatty acid component has a therapeutic effect.

8. A composition of claim 1 wherein the fatty acid component has a therapeutic effect while being in a complex with the agonist.

9. A composition of claim 1 wherein the fatty acid component has a therapeutic effect while not being in a complex with the agonist.

10. A composition of claim 1 wherein the fatty acid component is effective to reduce intraocular pressure when it is administered to the eye.

11. A composition of claim 1 wherein the fatty acid component is selected from the group consisting of prostanoids, derivatives thereof and mixtures thereof.

12. A composition of claim 1 wherein the fatty acid component is present in an amount effective to enhance the movement of the alpha-2-adrenergic agonist across a lipid membrane.

13. A composition of claim 1 wherein the fatty acid component enhances the movement of the agonist component across a biological membrane under physiological conditions.

14. A composition of claim 1 wherein the fatty acid component is effective to enhance the therapeutic effect provided by the agonist.

15. A composition of claim 1 wherein the complex is able to disassociate in a biological environment.

16. A composition of claim 1 which includes at least one additional agonist and the fatty acid is complexed with both the agonist and the additional agonist.

17. A composition of claim 1 which includes at least one additional fatty acid component and the agonist is complexed with both the fatty acid component and the additional fatty acid component.

18. A composition of claim 1 which further comprises a carrier.

19. A composition of claim 1 wherein the agonist comprises 5-bromo-6-(2-imidozolin-2-ylamino) quinoxaline; and
the fatty acid component is selected from the group consisting of docosahexanoic acids, linolenic acids, prostanoids, derivatives thereof and mixtures thereof.

20. An ophthalmically acceptable composition comprising:
a 5-bromo-6-(2-imidozolin-2-ylamino) quinoxaline; and
a linolenic acid component,
wherein the 5-bromo-6-(2-imidozolin-2-ylamino) quinoxaline forms a complex with the linoleic acid component, the complex substantially remains intact in an aqueous environment.

## Patentansprüche

1. Eine ophthalmologisch annehmbare Zusammensetzung, die einen alpha-2-andrenergen Agonisten, ausgewählt aus einer Chinoxalinverbindung, und eine Fettsäurekomponente, ausgewählt aus gesättigten Fettsäuren und ungesättigten Fettsäuren und Mischungen daraus, umfasst, worin die Fettsäurekomponente mit dem alpha-2-andrenergen Agonisten einen Komplex bildet und der Komplex in einer wässrigen Umgebung weitgehend intakt bleibt.

2. Zusammensetzung gemäß Anspruch 1, worin die Fettsäurekomponente in einer Menge vorhanden ist, die zur Erhöhung der Wirksamkeit des Agonisten relativ zur Wirksamkeit des alpha-2-andrenergen Agonisten ohne die Fettsäurekomponente wirksam ist.

3. Zusammensetzung gemäß Anspruch 2, worin die Chinoxalinkomponente ausgewählt ist aus Chinoxalin, (2-Imidozolin-2-ylamino)-chinoxalin, 5-Brom-6-(2-imidozolin-2-ylamino)-chinoxalin und Derivaten davon und Mischungen daraus.

4. Zusammensetzung gemäß Anspruch 1, worin die Fettsäurekomponente ausgewählt ist aus Fettsäuren mit ungefähr 12-16 Kohlenstoffatomen pro Molekül, Derivaten davon und Mischungen daraus.

5. Zusammensetzung gemäß Anspruch 1, worin die Fettsäurekomponente ausgewählt ist aus Docosahexansäuren, Derivaten davon und Mischungen daraus.

6. Zusammensetzung gemäß Anspruch 1, worin die Fettsäurekomponente ausgewählt ist aus Linolensäuren, Derivaten davon und Mischungen daraus.

7. Zusammensetzung gemäß Anspruch 1, worin die Fettsäurekomponente eine therapeutische Wirkung aufweist.

8. Zusammensetzung gemäß Anspruch 1, worin die Fettsäurekomponente eine therapeutische Wirkung aufweist, wenn sie in einem Komplex mit dem Agonisten vorliegt.

9. Zusammensetzung gemäß Anspruch 1, worin die Fettsäurekomponente eine therapeutische Wirkung aufweist, wenn sie nicht in einem Komplex mit dem Agonisten vorliegt.

10. Zusammensetzung gemäß Anspruch 1, worin die Fettsäurekomponente zur Verringerung des Augeninnendrucks wirksam ist, wenn sie am Auge verabreicht wird.

11. Zusammensetzung gemäß Anspruch 1, worin die Fettsäurekomponente ausgewählt ist aus Prostanoiden, Derivaten davon und Mischungen daraus.

12. Zusammensetzung gemäß Anspruch 1, worin die Fettsäurekomponente in einer Menge vorhanden ist, die zur Verstärkung der Bewegung des alpha-2-andrenergen Agonisten durch eine Lipidmembran wirksam st.

13. Zusammensetzung gemäß Anspruch 1, worin die Fettsäurekomponente die Bewegung der Agonistenkomponente durch eine biologische Membran unter physiologischen Bedingungen beschleunigt.

14. Zusammensetzung gemäß Anspruch 1, worin die Fettsäurekomponente wirksam ist zur Verstärkung der durch den Agonisten bereitgestellten therapeutischen Wirkung.

15. Zusammensetzung gemäß Anspruch 1, worin der Komplex in einer biologischen Umgebung dissoziieren kann.

16. Zusammensetzung gemäß Anspruch 1, die mindestens einen zusätzlichen Agonisten einschließt, und worin die Fettsäure mit sowohl dem Agonisten als auch dem zusätzlichen Agonisten komplexiert ist.

17. Zusammensetzung gemäß Anspruch 1, die mindestens eine zusätzliche Fettsäurekomponente einschließt, und worin der Agonist mit sowohl der Fettsäurekomponente als auch der zusätzlichen Fettsäurekomponente komplexiert ist.

18. Zusammensetzung gemäß Anspruch 1, die ferner einen Träger umfasst.

19. Zusammensetzung gemäß Anspruch 1, worin der Agonist 5-Brom-6-(2-Imidozolin-2-ylamino)-chinoxalin umfasst, und die Fettsäurekomponente ausgewählt ist aus Docosahexansäuren, Linolensäuren, Prostanoiden, Derivaten davon und Mischungen daraus.

20. Eine ophthalmologisch annehmbare Zusammensetzung, die ein 5-Brom-6-(2-Imidozolin-2-ylamino)-chinoxalin und eine Linolensäurekomponente umfasst, worin das 5-Brom-6-(2-Imidozolin-2-ylamino)-chinoxalin mit der Linolensäurekomponente einen Komplex bildet und der Komplex in einer wässrigen Umgebung weitgehend intakt bleibt.

## Revendications

1. Composition acceptable du point de vue ophtalmologique comprenant un agoniste alpha-2-adrénergique choisi parmi un composé quinoxaline et un composant d'acide gras choisi parmi les acides gras saturés, les acides gras insaturés et leurs mélanges, le composant d'acide gras formant un complexe avec l'agoniste alpha-2-adrénergique, le complexe restant essentiellement intact dans un environnement aqueux.

2. Composition selon la revendication 1, dans laquelle le composant d'acide gras est présent en une quantité efficace pour favoriser l'efficacité de l'agoniste par rapport à l'efficacité de l'agoniste alpha-2-adrénergique sans le composant d'acide gras.

3. Composition selon la revendication 2, dans laquelle le composant quinoxaline est choisi parmi la quinoxaline, la (2-imidozolin-2-ylamino)quinoxaline, la 5-bromo-6-(2-imidozolin-2-ylamino)quinoxaline et leurs dérivés et leurs mélanges.

4. Composition selon la revendication 1, dans laquelle le composant d'acide gras est choisi parmi les acides gras ayant d'environ 12 à environ 26 atomes de carbone par molécule, leurs dérivés et leurs mélanges.

5. Composition selon la revendication 1, dans laquelle le composant d'acide gras est choisi parmi les acides docosahexanoïques, leurs dérivés et leurs mélanges.

6. Composition selon la revendication 1, dans laquelle le composant d'acide gras est choisi parmi les acides linoléniques et leurs mélanges.

7. Composition selon la revendication 1, dans laquelle le composant d'acide gras a un effet thérapeutique.

8. Composition selon la revendication 1, dans laquelle le composant d'acide gras a un effet thérapeutique tout en étant dans un complexe avec l'agoniste.

9. Composition selon la revendication 1, dans laquelle le composant d'acide gras a un effet thérapeutique tout en n'étant pas dans un complexe avec l'agoniste.

10. Composition selon la revendication 1, dans laquelle le composant d'acide gras est efficace pour réduire la pression intraoculaire lorsqu'il est administré à l'oeil.

11. Composition selon la revendication 1, dans laquelle le composant d'acide gras est choisi parmi les prostanoïdes, leurs dérivés et leurs mélanges.

12. Composition selon la revendication 1, dans laquelle le composant d'acide gras est présent en une quantité efficace pour favoriser le mouvement de l'agoniste alpha-2-adrénergique au niveau d'une membrane de lipide.

13. Composition selon la revendication 1, dans laquelle le composant d'acide gras favorise le mouvement du composant agoniste à travers une membrane biologique sous des conditions physiologiques.

14. Composition selon la revendication 1, dans laquelle le composant d'acide gras est efficace pour augmenter l'effet thérapeutique fourni par l'agoniste.

15. Composition selon la revendication 1, dans laquelle le complexe est capable de se dissocier dans un environnement biologique.

16. Composition selon la revendication 1, qui comprend au moins un agoniste supplémentaire et l'acide gras est complexé tant avec l'agoniste qu'avec l'agoniste supplémentaire.

17. Composition selon la revendication 1, qui comprend au moins un composant d'acide gras supplémentaire et l'agoniste est complexé tant avec le composant d'acide gras qu'avec le composant d'acide gras supplémentaire.

18. Composition selon la revendication 1, qui comprend, en outre, un véhicule.

19. Composition selon la revendication 1, dans laquelle l'agoniste comprend la 5-bromo-6-(2-imidozolin-2-ylamino)quinoxaline ; et
le composant d'acide gras est choisi parmi les acides docosahexanoïques, les acides linoléniques, les prostanoides, leurs dérivés et leurs mélanges.

20. Composition acceptable du point de vue ophtalmologique comprenant :
une 5-bromo-6-(2-imidozolin-2-ylamino)quinoxaline ; et
un composant d'acide linolénique,
dans laquelle la 5-bromo-6-(2-imidozolin-2-ylamino) quinoxaline forme un complexe avec le composant d'acide linolénique, le complexe restant essentiellement intact dans un environnement aqueux.
